# EUROPEAN PATENT APPLICATION

(11) **EP 4 537 762 A1**
(43) Date of publication of application: **16.04.2025**
(21) Application number: 22945916.9
(22) Date of filing: 31.08.2022
(51) Int. Cl.: A61B 7/04

(54) **SOUND RECORDING DEVICE, INFORMATION PROCESSING SYSTEM, SOUND RECORDING METHOD, AND PROGRAM**

(30) Priority: 07.06.2022 JP 2022092105
(71) Applicant: Suntory Holdings Limited, Osaka 530-8203 (JP)
(72) Inventor: KUTSUMI Yuka, Kyoto 6190284 (JP); KANEGAWA Norimasa, Kyoto 6190284 (JP)
(74) Representative: Vossius & Partner Patentanwälte Rechtsanwälte mbB
(86) International application number: PCT/JP2022/032848
(87) International publication number: WO 2023/238420

(57) **Abstract**

A problem to be solved by conventional sound recording apparatuses is how to make it possible to acquire high-quality recording results. It is possible to acquire a high-quality recording result by using a sound recording apparatus that has a microphone accommodated in a housing thereof and is used to record a user's abdominal sounds in a state where a sound collecting portion of the housing near the microphone is in contact with the user's abdomen, the sound recording apparatus including: an environment detection unit 41 that detects at least one of an inappropriate environment that is inappropriate for recording the abdominal sounds and an appropriate environment that is appropriate for recording the abdominal sounds; and a sound recording processing unit 51 that performs processing related to sound recording based on a result of detection by the environment detection unit 41.

## Description

### Technical Field

The present invention relates to a sound recording apparatus, an information processing system, a recording method, and a program that can be used when recording abdominal sounds by contacting a portion near a built-in microphone with a human abdomen.

### Background Art

In recent years, users have become increasingly health conscious. Under such circumstances, various apparatuses and services are now available to support health and lifestyle habit information. For example, Patent Document 1, listed below, describes the configuration of a health management support system that is configured to acquire and record a user's health management information and output health management-related messages to the user based on the contents of the health management information.

The intestines (small intestine and large intestine) are known as one of the most important organs for digestion and absorption of nutrients and for symbiosis with gut bacteria. In recent years, there has been an increasing need for gut health, which is reflected in the term "gut health", for example.

Patent Document 2, listed below, describes an analyzing apparatus that evaluates gastrointestinal motility for the purpose of diagnosing intestinal diseases based on acoustic features extracted using machine learning techniques from sound recording data acquired by a non-contact microphone.

In addition, Non-Patent Document 1, listed below, describes the recording of gut sounds over a relatively long period of time using a low-cost, simple piezoelectric acoustic sensing device for use in the diagnosis of digestive diseases.

### Citation List

### Patent Documents

Patent Document 1: JP 2017-041035A
Patent Document 2: WO 2019/216320A1

### Non-Patent Document

Non-Patent Document 1: Du, X. et al. 2018 Bowel Sounds Identification and Migrating Motor Complex Detection with Low-Cost Piezoelectric Acoustic Sensor. Sensors. 18 (12), 4240

### Summary of Invention

### Technical Problem

If it becomes possible to record a user's abdominal sounds and acquire information that is useful for managing the user's health, it will be possible to contribute to the user's health management. When recording abdominal sounds, it is desirable to acquire a higher quality recording result.

Therefore, the present invention aims to provide a sound recording apparatus, an information processing system, a sound recording method, and a program that can acquire a high-quality recording result of abdominal sounds.

### Solution to Problem

A sound recording apparatus according to a first aspect of the present invention is a sound recording apparatus that has a microphone accommodated in a housing thereof and is used to record a user's abdominal sounds in a state where a sound collecting portion of the housing near the microphone is in contact with the user's abdomen, the sound recording apparatus including: an environment detection unit that detects at least one of an inappropriate environment that is inappropriate for recording the abdominal sounds and an appropriate environment that is appropriate for recording the abdominal sounds; and a sound recording processing unit that performs processing related to sound recording based on a result of detection by the environment detection unit.

With such a configuration, it is possible to acquire a high-quality recording result of abdominal sounds.

A sound recording apparatus according to a second aspect of the present invention is the sound recording apparatus according to the first aspect of the invention, wherein the environment detection unit acquires information regarding a pressing force of the sound recording apparatus against the abdomen based on sound information acquired through sound recording performed using the microphone, and performs the detection based on the acquired information.

With such a configuration, it is possible to acquire a high quality recording result of abdominal sounds that is recorded with a pressing force that is not inappropriate.

A sound recording apparatus according to a third aspect of the present invention is the sound recording apparatus according to the second aspect of the invention, wherein the environment detection unit detects noise in the sound information, and acquires information regarding the pressing force based on a result of the detection.

With such a configuration, it is possible to acquire information regarding the pressing force with higher accuracy.

A sound recording apparatus according to a fourth aspect of the present invention is the sound recording apparatus according to the second or third aspect of the invention, wherein the environment detection unit acquires information regarding a current pressing force based on past sound information and current sound information, and performs the detection regarding the current pressing force based on the acquired information.

With such a configuration, even in a situation where the pressing force may change from time to time, it is easier to continue sound recording with a pressing force that is not inappropriate.

A sound recording apparatus according to a fifth aspect of the present invention is any one of the first to fourth aspects of the invention, wherein the environment detection unit performs the detection based on a sound-to-noise ratio between abdominal sounds and noise in the sound information acquired through sound recording performed using the microphone.

With such a configuration, it is possible to acquire information regarding the pressing force with higher accuracy.

A sound recording apparatus according to a sixth aspect of the present invention is any one of the first to fifth aspects of the invention, wherein the sound recording apparatus is a smartphone in which a phone case is attachable to the housing, and the environment detection unit acquires information regarding whether or not a phone case is attached to the housing, and performs the detection based on the acquired information.

With such a configuration, it is possible to acquire information regarding the presence or absence of a phone case, which may affect the quality of the recording of abdominal sounds.

A sound recording apparatus according to a seventh aspect of the present invention is the sound recording apparatus according to the sixth aspect of the invention, wherein the environment detection unit detects electromagnetic noise contained in sound information acquired through sound recording performed using the microphone, and acquires information regarding whether or not a phone case is attached to the housing based on a result of detection of the electromagnetic noise.

With such a configuration, it is possible to acquire information regarding the presence or absence of a phone case with higher accuracy.

A sound recording apparatus according to an eighth aspect of the present invention is the sound recording apparatus according to the sixth or seventh aspect of the invention, wherein the environment detection unit acquires information regarding whether or not a phone case is attached to the housing when a predetermined condition regarding quality of sound information acquired through sound recording performed using the microphone is satisfied.

With such a configuration, it is possible to acquire information regarding the presence or absence of a phone case with higher accuracy.

A sound recording apparatus according to a ninth aspect of the present invention is any one of the sixth to eighth aspects of the invention, wherein when a fact that a phone case is attached to the housing is detected, the sound recording processing unit provides a notification to the user of the sound recording apparatus to prompt the user to remove the phone case.

With such a configuration, it is possible to acquire a high-quality recording result of abdominal sounds in a state where a phone case, which may affect the quality of the recording of abdominal sounds, is removed.

A sound recording apparatus according to a tenth aspect of the present invention is any one of the first to ninth aspects of the invention, wherein the environment detection unit performs the detection based on information regarding an orientation of the sound recording apparatus.

With such a configuration, it is possible to acquire a high-quality recording result of abdominal sounds recorded in state where the sound recording apparatus is in an orientation that is not inappropriate.

A sound recording apparatus according to an eleventh aspect of the present invention is the sound recording apparatus according to the tenth aspect of the invention, wherein the sound recording apparatus is a smartphone whose housing has a plate shape and a touch panel is disposed on one surface thereof, the sound collecting portion is located at an end portion of the housing, and the environment detection unit performs the detection when the user is in a sitting position and the sound collecting portion is in contact with the abdomen, based on information regarding whether an angle between the touch panel and a line perpendicular to a plane that is in contact with a surface of the abdomen at a contact site is within a predetermined angle range.

With such a configuration, it is possible to acquire a high-quality recording result of abdominal sounds recorded in state where the sound recording apparatus is in an orientation that is not inappropriate.

A sound recording apparatus according to a twelfth aspect of the present invention is any one of the first to eleventh aspects of the invention, wherein the sound recording processing unit is configured to output predetermined guidance information from the sound recording apparatus when a fact that the environment is the inappropriate environment or a fact that the environment is not the appropriate environment is detected.

With such a configuration, it is possible to prevent abdominal sounds from being recorded in an inappropriate state.

A sound recording apparatus according to a thirteenth aspect of the present invention is any one of the first to eleventh aspects of the invention, wherein the sound recording processing unit is configured to perform predetermined processing to carry out sound recording using the microphone or to use sound information acquired through sound recording performed using the microphone when a fact that the environment is the appropriate environment or a fact that the environment is not the inappropriate environment is detected.

With such a configuration, it is possible to acquire and use a high-quality recording result of abdominal sounds.

A sound recording apparatus according to a fourteenth aspect of the present invention is the sound recording apparatus according to the thirteenth aspect of the invention, wherein the predetermined processing is to transmit the sound information to an external apparatus.

With such a configuration, it is possible to use a high-quality recording result of abdominal sounds in an external apparatus.

An information processing system according to a fifteenth aspect of the present invention is an information processing system including : the sound recording apparatus according to claim 1; and an information processing apparatus that can communicate with the sound recording apparatus, wherein the sound recording apparatus includes a terminal output unit that outputs sound information acquired by recording a user's abdominal sounds using the microphone, and the information processing apparatus includes: a sound information acquisition unit that acquires the sound information; a gut score acquisition unit that acquires a gut score related to the user's gut condition, using input information containing the sound information acquired by the sound information acquisition unit and learning information prepared in advance; and a gut score output unit that outputs the gut score acquired by the gut score acquisition unit.

With such a configuration, it is possible to output a gut score related to the user's gut condition, using a high-quality recording result of abdominal sounds.

A sound recording method according to a sixteenth aspect of the present invention is a sound recording method for recording a user's abdominal sounds using the sound recording apparatus according to any one of the first to fifteenth aspects of the invention, including: a first step of bringing the sound collecting portion into contact with the user's abdomen in a state where the user is in a sitting position; a second step of causing the environment detection unit to perform the detection in a state where the sound collecting portion is in contact with the abdomen; and a third step of causing the sound recording processing unit to perform processing related to the sound recording based on a result of detection by the environment detection unit.

With such a configuration, it is possible to acquire a high-quality recording result of abdominal sounds.

### Advantageous Effects of Invention

With the sound recording apparatus and so on according to the present invention, it is possible to acquire a high-quality recording result of abdominal sounds.

### Brief Description of Drawings

FIG. 1 is a diagram showing an overview of an information processing system according to one embodiment of the present invention.
FIG. 2 is a block diagram for an information processing apparatus according to the same.
FIG. 3 is a block diagram for a portable information terminal according to the same.
FIG. 4 is a perspective view showing the portable information terminal according to the same.
FIG. 5 is a diagram illustrating abdominal sound recording performed with the portable information terminal according to the same.
FIG. 6 is a diagram showing an example of a situation in which a user records abdominal sounds with the portable information terminal according to the same.
FIG. 7 is a graph showing an example of a relationship between a pressing force and the volume of noise in sound information.
FIG. 8 is a flowchart showing examples of operations of the information processing apparatus according to the same.
FIG. 9 is a flowchart showing examples of operations of the information processing apparatus according to the same.
FIG. 10 is a first diagram showing specific examples of screen transitions of the portable information terminal according to the same.
FIG. 11 is a second diagram showing specific examples of screen transitions of the portable information terminal according to the same.
FIG. 12 is an overview diagram for a computer system according to the above embodiments.
FIG. 13 is a block diagram for the computer system according to the same.

### Description of Embodiments

Hereinafter, embodiments of a sound recording apparatus and so on will be described with reference to the drawings. Note that in the embodiments, constituent elements with the same reference signs perform similar operations, and therefore, repeated descriptions thereof may be omitted.

In the following description, the terms used are generally defined as follows. Note that the meanings of these terms should not always be interpreted as given here, but, for example, where they are described individually below, they should be interpreted in the light of that description.

Abdominal sounds refer to sounds emitted from a user's abdomen. Abdominal sounds can include, for example, gut sounds emitted from the intestines. Abdominal sounds may also include sounds caused by blood flow in the abdomen (for example, abdominal aortic sounds) and sounds emitted from organs such as the stomach.

Sound information refers to information acquired based on abdominal sounds. Sound information may be the recorded abdominal sound data itself, or may be data obtained by processing or editing the recorded data, or the like.

Lifestyle information is information regarding a user's living status. The living status may include various elements related to a user's actions, conditions, and so on. Lifestyle information may include, for example, excretion-related information regarding a user's excretion status, dietary information regarding a user's dietary status, situational activities related to lifestyle habits other than the dietary status (referring to actions and behaviors that the user repeatedly performs in their daily life), and so on. However, lifestyle information is not limited to the above examples, and may include information regarding other elements, or may include information regarding only some of these elements. Note that each piece of information regarding the elements of lifestyle information (excretion-related information, dietary information, activity status information, and so on) may include information regarding smaller elements.

Basic information is information regarding a user's morphology and characteristics. For example, basic information may include various information such as the user's height, weight, age, sex, body fat percentage, muscle mass, pulse rate, respiratory rate, and blood pressure. Basic information may also include the user's responses to a questionnaire, information generated based on the responses, information regarding the user's medical history, and so on. For example, responses to questionnaire items used to diagnose people with intestinal disorders can objectively represent the user's constitution. Basic information is, for example, information that is input in advance by the user, but this is not essential, and basic information may be, for example, information acquired by a wearable terminal or the like worn by the user, or information acquired from an external database or the like in which user information is recorded.

An identifier for an item is a character or code that uniquely identifies the item. An identifier is, for example, an ID, but there is no limitation on the type thereof as long as it is information that can identify the corresponding item. That is to say, an identifier may be the name of the item it indicates, or may be a combination of codes that uniquely correspond to the item.

Acquisition may include acquiring information input by a user or the like, or may include acquiring information stored in the apparatus itself or another apparatus (which may be information stored in advance or information generated by information processing performed in the apparatus). Acquiring information stored in another apparatus may include acquiring information stored in the other apparatus via an API or the like, or may include acquiring the contents of a document file (including the contents of a web page) provided by the other apparatus. It may also include acquiring information in a different format from the original information, such as acquiring information by performing optical character reading on an image.

In addition, so-called machine learning techniques may be used to acquire information. Machine learning techniques can be used in the following manner, for example. That is to say, a learning model (learning information) that receives a specific type of input information as input and outputs the type of output information that is desired to be acquired is formed using machine learning techniques. For example, two or more sets of input information and output information are prepared in advance, and the two or more sets of information are provided to a module for forming a machine learning model to form a learning model, and the learning model thus formed is accumulated in a storage unit. The learning model can also be called a classifier. Examples of machine learning techniques include deep learning, random forest, SVM, and so on, and there is no limitation. In addition, functions in various machine learning frameworks such as fastText, tinySVM, random forest, and TensorFlow, and various existing libraries, can be used for machine learning.

In addition, the learning model is not limited to one acquired by machine learning. The learning model may be, for example, a table indicating the correspondence between input vectors that are based on input information or the like and pieces of output information. In this case, the piece of output information corresponding to the feature vector based on input information may be acquired from the table, or a vector approximating the feature vector based on input information may be generated using two or more input vectors in the table and parameters that weight the input vectors, and the final output information may be acquired using the pieces of output information and parameters corresponding to the input vectors used in the generation. The learning model may be, for example, a function that represents the relationship between input vectors that are based on input information or the like, and pieces of information for generating output information. In this case, for example, information corresponding to a feature vector that is based on input information may be obtained using the function, and output information may be obtained using the obtained information.

The "output" of information is a concept that encompasses displaying on a display screen, projection using a projector, printing by a printer, the output of a sound, transmission to an external apparatus, accumulation on a recording medium, delivery of a processing result to another processing apparatus or another program, and the like. Specifically, this concept includes, for example, enabling the information to be displayed on a web page, sending the information as an e-mail or the like, outputting information for printing, and so on.

The "acceptance" of information is a concept that includes, for example, acceptance of information input from an input device such as a keyboard, a mouse, or a touch panel, reception of information transmitted from another apparatus or the like via a wired or wireless communication network, or acceptance of information read out from a recording medium such as an optical disk, a magnetic disk, or a semiconductor memory.

### Embodiments

In the present embodiment, a portable information terminal is used as a sound recording apparatus. The sound recording apparatus is configured to detect an inappropriate environment or appropriate environment when recording abdominal sounds, and to perform processing according to the result of the detection. Hereinafter, an information processing system 1 formed using such a sound recording apparatus will be described.

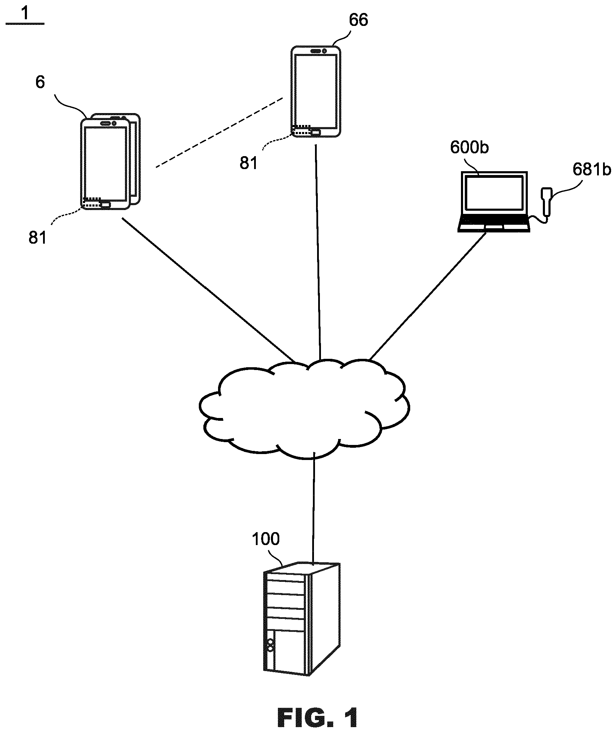

FIG. 1 is a diagram showing an overview of the information processing system 1 according to one embodiment of the present invention.

In the present embodiment, the information processing system 1 includes an information processing apparatus 100 and a portable information terminal 6 (an example of a sound recording apparatus). The information processing apparatus 100 and the portable information terminal 6 are capable of communicating with each other via a network such as a local area network or the Internet. Note that the configuration of the information processing system 1 is not limited to this example. There is no limitation on the number of apparatuses included in the information processing system 1, and other apparatuses may also be included in the information processing system 1.

A user of the information processing system 1 can use the information processing system 1 by using the portable information terminal 6. The portable information terminal 6 may also be referred to as a terminal apparatus. In the present embodiment, so-called smartphones, each having a built-in microphone 81, are shown as portable information terminals 6. Note that terminals that can be used as portable information terminals 6 are not limited to such smartphones. For example, a portable information terminal 600b that is a personal computer (PC) such as a laptop computer may be used, or other apparatuses such as a tablet-type information terminal apparatus may be used. In addition, an external microphone 681b may be used as the microphone. In the following example, it is assumed that a smartphone is used as a portable information terminal 6, but the present invention is not limited to this example.

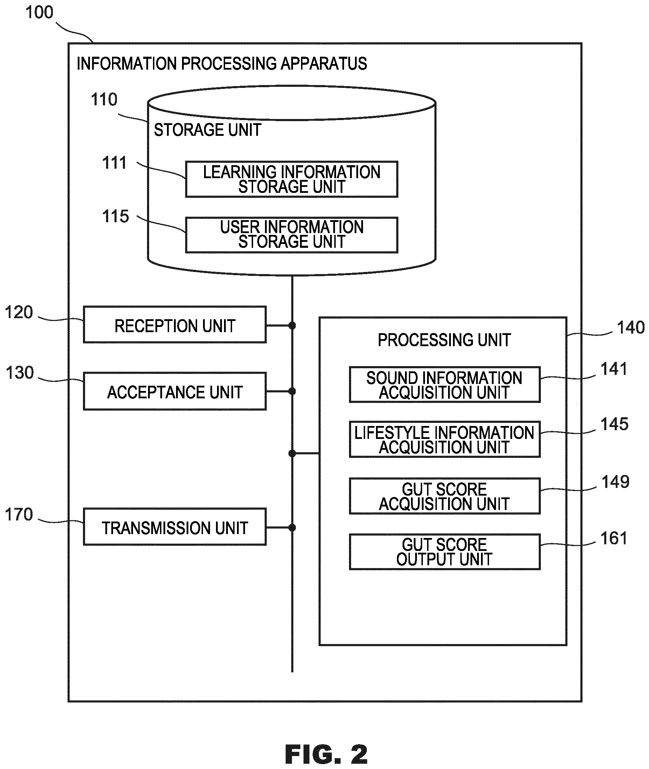

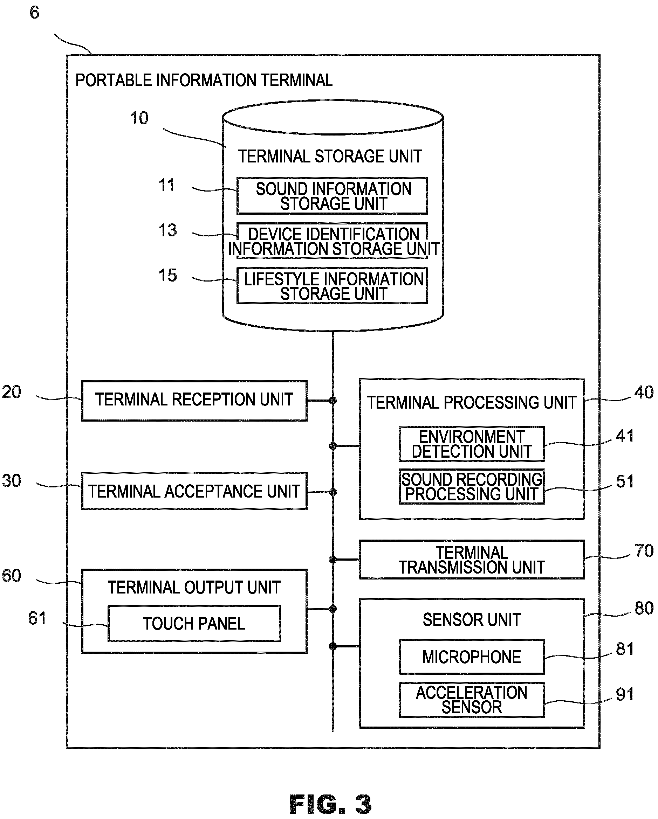

FIG. 2 is a block diagram for the information processing apparatus 100 according to the same. FIG. 3 is a block diagram for the portable information terminal 6 according to the same.

As shown in FIG. 2, the information processing apparatus 100 includes a storage unit 110, a reception unit 120, an acceptance unit 130, a processing unit 140, and a transmission unit 170. The information processing apparatus 100 is, for example, a server apparatus.

The storage unit 110 includes a learning information storage unit 111 and a user information storage unit 115.

The learning information storage unit 111 stores learning information acquired in advance. In the present embodiment, learning information is created by setting learning input information including sound information as input information, and predetermined output index values related to gut activity conditions as output information. For example, the learning information is generated by using a so-called machine learning technique as described above. For example, the learning information is generated by the information processing apparatus 100, but there is no such limitation. That is to say, the learning information generated by an apparatus other than the information processing apparatus 100 may be stored in the learning information storage unit 111.

The user information storage unit 115 stores user information. In the present embodiment, the user information is information in which user identifiers, which are identifiers that respectively identify users who use the information processing system 1, and pieces of information regarding the users are associated with each other. The user information may include various kinds of information. Examples of the user information may include information transmitted from portable information terminals 6 used by the users, information regarding the users acquired by the information processing apparatus 100 in a manner described below, and so on. Examples of the information transmitted from the portable information terminals 6 used by the users include basic information regarding the users, sound information, lifestyle information, and so on.

The reception unit 120 receives information transmitted from other apparatuses. The reception unit 120 stores the received information in the storage unit 110, for example. In the present embodiment, the users input information using the portable information terminals 6, for example, to transmit the information to the information processing apparatus 100. The reception unit 120 can store the pieces of transmitted information in the storage unit 110 in association with the user identifiers. In addition, as will be described later, in the present embodiment, sound information transmitted from each portable information terminal 6 can be received and stored in the storage unit 110 in association with a user identifier. When receiving such information from a portable information terminal 6, the reception unit 120 is able to identify the user identifier of the user related to the transmission based on the transmitted information.

The acceptance unit 130 accepts information input using an input means (not shown) connected to the information processing apparatus 100. The acceptance unit 130 stores the accepted information in the storage unit 110, for example. Note that any input means, such as a numeric keypad, a keyboard, a mouse, a menu screen, or the like, may be employed.

The processing unit 140 includes a sound information acquisition unit 141, a lifestyle information acquisition unit 145, a gut score acquisition unit 149, and a gut score output unit 161. The processing unit 140 performs various kinds of processing. The various kinds of processing are, for example, processing performed by the units included in the processing unit 140 as described below.

The sound information acquisition unit 141 acquires sound information of one user. In the present embodiment, the sound information acquisition unit 141 acquires the sound information transmitted from the user's portable information terminal 6 and received by the reception unit 120.

The lifestyle information acquisition unit 145 acquires lifestyle information. The lifestyle information acquisition unit 145 acquires information stored in the user information storage unit 115, for example. In addition, the lifestyle information acquisition unit 145 may acquire the user's basic information.

The gut score acquisition unit 149 acquires a gut score related to the user's gut condition, using input information containing the sound information acquired by the sound information acquisition unit 141 and learning information prepared in advance. In the present embodiment, the gut score acquisition unit 149 acquires predetermined output index values using the learning information stored in the learning information storage unit 111, and acquires the gut score using this output index values. For example, at least one of the bowel movement condition and the number of gut peristaltic movements per unit time is used. The number of gut peristaltic movements may be referred to as the frequency at which gut sounds at or above a predetermined level occur (hereinafter, may simply be referred to as the frequency of gut sounds).

Specifically, the gut score acquisition unit 149 acquires the gut score in the following manner, for example. The gut score acquisition unit 149 uses the sound information acquired by the sound information acquisition unit 141 and the learning information to acquire a gut condition score related to a bowel movement condition (normal, diarrhea, constipation, etc.) and a gut movement score related to gut peristaltic movements (frequency, etc.). In the present embodiment, the gut score acquisition unit 149 acquires output index values by inputting the sound information acquired by the sound information acquisition unit 141 to learning information, using a machine learning technique. Thereafter, gut-related scores (a gut condition score and a gut movement score) are acquired based on the output index values. The gut-related scores can be acquired as gut scores.

Here, in the present embodiment, the sound information input to the gut score acquisition unit 149 is a spectrum image that represents in a predetermined form the results of analyzing audio data (which may be processed) acquired by recording abdominal sounds, using the Fourier transform or the fast Fourier transform. Note that the sound information may be audio data (which may be processed) itself, or may be data converted into another format. The sound information to be input to the gut score acquisition unit 149 may be prepared in the processing unit 140 using sound information transmitted to the information processing apparatus 100 from a device that has recorded abdominal sounds, for example. Note that sound information that is a spectrum image may be prepared in advance in a device other than the information processing apparatus 100, such as a device that has recorded abdominal sounds, and the sound information may be transmitted to the information processing apparatus 100.

Note that the learning information generated for each of several types by which users can be classified by using user information may be stored in the learning information storage unit 111 in association with identifiers that identify the types. In this case, the gut score acquisition unit 149 may be configured to acquire learning information of a type corresponding to an identifier identified based on user information from the learning information storage unit 111, and acquire the gut-related scores using the learning information. This makes it possible to acquire output results with higher accuracy.

Based on information different from sound information, such as various output index values such as excretion, dietary, activity status, and various kinds of lifestyle information, the gut score acquisition unit 149 may acquire scores for the elements and may combine these scores to acquire the gut score. The scores for such elements may be acquired based on whether or not the output index values and the contents of the various kinds of lifestyle information satisfy predetermined conditions, for example. The gut score acquisition unit 149 uses the acquired gut-related scores and the scores for other elements to acquire a gut score. For example, the gut score may be acquired by calculating the gut-related scores and other scores by applying them to a predetermined calculation formula, such as by adding or multiplying them in a predetermined manner. The gut score may also be acquired using a learning model formed using a machine learning technique so as to output the gut score in response to the input scores. The scores may be normalized as necessary before being used to acquire the gut score.

The gut score acquisition unit 149 accumulates the acquired gut score in the user information storage unit 115 in association with the user identifier.

The gut score output unit 161 outputs the gut score acquired by the gut score acquisition unit 149. In the present embodiment, the gut score output unit 161 outputs information such as the gut score by transmitting it to the portable information terminal 6, but there is no such limitation. For example, the gut score output unit 161 may output the gut score by displaying it as characters or images on a display included in the information processing apparatus 100.

The transmission unit 170 transmits information to other apparatuses included in the information processing system 1 via the network. For example, the transmission unit 170 transmits information to the portable information terminal 6. In other words, for example, the transmission unit 170 outputs information to the portable information terminal 6.

Next, the configuration of the portable information terminal 6 will be described. The portable information terminal 6 is a smartphone. In the information processing system 1, the portable information terminal 6 is used as a sound recording apparatus for recording the user's abdominal sounds, and is also used as a terminal for outputting information regarding the gut score acquired by the information processing apparatus 100 so that the user can check it.

As shown in FIG. 3, the portable information terminal 6 includes a terminal storage unit 10, a terminal reception unit 20, a terminal acceptance unit 30, a terminal processing unit 40, a terminal output unit 60, a terminal transmission unit 70, and a sensor unit 80.

The terminal storage unit 10 includes a sound information storage unit 11, a device identification information storage unit 13, and a lifestyle information storage unit 15.

The sound information storage unit 11 accumulates sound information (which may also be referred to as recorded sound information) recorded using the sensor unit 80 of the portable information terminal 6. Sound information is transmitted to the information processing apparatus 100 by the terminal transmission unit 70, and sound information may be deleted from the sound information storage unit 11 when the transmission is complete, or retained until a specified period of time has elapsed, or retained permanently until a deletion operation is performed by the user.

The device identification information storage unit 13 stores device identification information. For example, the device identification information is, for example, information such as an identifier that can identify the model (type) of the portable information terminal 6, but there is no such limitation. For example, device identification information may be an identifier that can identify the model of a device included in the portable information terminal 6 (for example, a hardware module or the like included in the microphone 81). It may be possible that different information cannot be written to the device identification information storage unit 13.

The lifestyle information storage unit 15 accumulates lifestyle information. Lifestyle information is transmitted to the information processing apparatus 100 by the terminal transmission unit 70, and lifestyle information may be deleted from the lifestyle information storage unit 15 when the transmission is complete, or retained until a specified period of time has elapsed, or retained permanently until a deletion operation is performed by the user.

The lifestyle information stored in the lifestyle information storage unit 15 may include information input by the user or information based on the same. These pieces of information may be, for example, information accepted by the terminal acceptance unit 30, or information acquired by the terminal processing unit 40 by performing calculations or the like based on the information accepted by the terminal acceptance unit 30.

The lifestyle information stored in the lifestyle information storage unit 15 may include a measurement value or information based on the same. For example, these pieces of information may be information measured by the sensor unit 80, or information acquired by the terminal processing unit 40 by performing calculations, etc. based on the information measured by the sensor unit 80. These pieces of information may also be information measured by a sensor apparatus communicatively connected to the portable information terminal 6 and transmitted to the portable information terminal 6.

The terminal reception unit 20 receives information transmitted from the information processing apparatus 100 and other apparatuses via the network. The terminal reception unit 20 accumulates the received information, for example, in the terminal storage unit 10 so that the terminal processing unit 40 and so on can acquire it.

The terminal acceptance unit 30 accepts various operations input to the portable information terminal 6 by the user who uses the portable information terminal 6. For example, the operations are performed using a touch panel 61 or other operation buttons, but there is no such limitation. For example, the terminal acceptance unit 30 may accept an input operation by voice input through the microphone 81. The terminal acceptance unit 30 may be regarded as accepting the information received by the terminal reception unit 20 as information input to the portable information terminal 6. That is to say, input of information to the portable information terminal 6 may be interpreted as meaning that information is indirectly input to the portable information terminal 6 by the user via another apparatus. In addition, it may be considered as inputting information to the portable information terminal 6 when the user allows information to be given to the portable information terminal 6 by, for example, executing a program that automatically generates information or giving various kinds of information to the program to make it function.

The terminal processing unit 40 performs various kinds of processing. Examples of the various kinds of processing include processing performed by the units included in the processing unit 140 using the units included in the portable information terminal 6 as appropriate, as described below.

In the present embodiment, the terminal processing unit 40 has an environment detection unit 41 and a sound recording processing unit 51 for performing control related to recording of the user's abdominal sounds.

The environment detection unit 41 detects the environment in which abdominal sounds are recorded. In the present embodiment, the environment detection unit 41 detects that the environment in which the portable information terminal 6 is located is an appropriate environment for recording abdominal sounds. In other words, the environment detection unit 41 detects that the environment in which the portable information terminal 6 is located is not an appropriate environment. The environment detection unit 41 may be configured to detect that the environment in which the portable information terminal 6 is located is an inappropriate environment, which is inappropriate for sound recording. The result of the detection is used in the sound recording processing unit 51.

In the following description, being an appropriate environment means not being an inappropriate environment, and not being an inappropriate environment means being an appropriate environment. However, there is no such limitation. That is to say, not being an appropriate environment does not necessarily mean being an inappropriate environment, and not being an inappropriate environment does not necessarily mean being an appropriate environment. There may be cases where the environment is not an inappropriate environment, but also not an appropriate environment. In this case, the environment detection unit 41 may be configured to be able to detect that the environment in which the portable information terminal 6 is located is both an appropriate environment and an inappropriate environment.

For example, an appropriate environment for recording abdominal sounds is an environment in which the volume of noise other than abdominal sounds in the recorded sound information is low. It can be said that an appropriate environment is an environment in which sound information with a low signal-to-noise ratio can be recorded. Examples of noise may include sounds (ambient noise) generated around the portable information terminal 6 recorded by the microphone 81, electromagnetic noise, and sounds (which may also be referred to as ambient noise) generated due to changes in the contact state between the portable information terminal 6 and other objects (rubbing, contact and separation, etc.). That is to say, an appropriate environment may be an environment in which the contact state between the portable information terminal 6 and other objects does not change, an environment in which electromagnetic noise is unlikely to be generated or recorded, or an environment in which sounds generated around the portable information terminal 6 are quiet or unlikely to be recorded.

Note that the environment detection unit 41 is configured to be able to specify noise in the sound information by a predetermined method as described below. That is to say, in the present embodiment, the environment detection unit 41 is configured to specify noise in the sound information based on, for example, the regularity of sounds. For example, it is possible to detect whether or not the sounds of interest are sounds with regularity, and to determine whether or not the sounds are noise based on the result. The detection of whether or not sounds have regularity may be performed, for example, based on the result of the detection of the intervals between the sounds, or may be performed based on the result of the Fourier analysis on the sound information. In general, gut sounds in abdominal sounds often occur without regularity, so by specifying sounds that have a regularity as noise, gut sounds can be effectively distinguished from noise in the sound information. Note that the method of specifying noise in the sound information is not limited to this example, and various methods can be used. For example, sounds in a predetermined frequency band may be regarded as noise, or noise may be specified using learning information that can extract noise from sound information. The environment detection unit 41 may be configured to use a combination of these methods. Note that the sound information may be transmitted to another apparatus (for example, the information processing apparatus 100 or the like) as appropriate, and specific processing related to specifying noise may be performed by the other apparatus. In this case, the result of the processing by the other apparatus may be received by the terminal reception unit 20, and the environment detection unit 41 may be configured to specify noise based on the received result of the processing, or to perform environment-related detection based on the received result of the noise specification.

The environment detection unit 41 performs detection with respect to the environment in which sound recording is carried out, based on information acquired using the sensor unit 80, as described below. The detection may be performed before the sound recording is carried out, while the sound recording is carried out, or after the sound recording is carried out.

The sound recording processing unit 51 performs processing related to recording of abdominal sounds in accordance with the result of the detection by the environment detection unit 41. Examples of processing related to recording include processing related to triggering sound recording and processing related to the execution of recording during sound recording. Examples of processing related to triggering sound recording include starting recording, and enabling or disabling the acceptance of a user operation for starting recording. Examples of processing related to the execution of recording during sound recording include the interruption, termination, and suspension (interruption, etc.) of sound recording, the enabling or disabling the acceptance of user operations for the purpose of, for example, interrupting sound recording, and the extension or reduction of a specified sound recording period. Processing related to sound recording may also be processing performed on the recorded results (sound information), such as discarding the sound information, associating the sound information with information regarding acceptance or rejection, associating the sound information with evaluation information regarding quality (such as information regarding the volume of noise), or transmitting the sound information to an external apparatus such as information processing apparatus 100. Processing related to sound recording may also include notifying the user of information regarding whether or not the volume of ambient noise, the user's posture, the orientation of the portable information terminal 6, and so on are appropriate during the sound recording, and information indicating the degree of these elements. Notification to the user may be said to be outputting guidance information that alerts the user or guides the user on how to improve the situation.

Although details will be described later, the sound recording processing unit 51 can be configured to perform the following processing, for example. That is to say, the sound recording processing unit 51 may be configured to output predetermined guidance information from the portable information terminal 6, for example, when the environment detection unit 41 detects that the environment is an inappropriate environment or not an appropriate environment. The guidance information can be provided by, for example, the terminal output unit 60 displaying it on the touch panel 61 or outputting it from a speaker or the like (not shown). The guidance information may be a display warning the user that the environment is not suitable for sound recording, a display providing guidance on how to improve the environment, or the like. It is preferable that the guidance information output be information related to the cause of the environment being unsuitable for sound recording. This can prevent sound recording from being performed in situations where it is unlikely that a high quality abdominal sound recording will be acquired. By modifying the environment based on the guidance information, the user can easily acquire a high quality abdominal sound recording. It can be said that these kinds of processing performed by the sound recording processing unit 51 are performed to acquire sound information in an appropriate environment.

In addition, the sound recording processing unit 51 may be configured to perform predetermined processing to execute recording using the microphone 81 or to use the sound information obtained by recording using the microphone 81 when the environment detection unit 41 detects that the environment is an appropriate environment or not an inappropriate environment during recording. For example, the sound recording processing unit 51 may execute recording during sound recording for a predetermined period of time as the predetermined processing. As a result, it is possible to reliably acquire an abdominal sound recording that is considered to be of high quality. In addition, in order to use the sound information acquired by sound recording, the sound information may be transmitted to the information processing apparatus 100. As a result, the information processing apparatus 100 can acquire a reliable gut score by using an abdominal sound recording that is considered to be of high quality. When transmitting sound information, the user's lifestyle information, basic information, etc. may be transmitted together with the sound information.

The terminal output unit 60 includes, for example, the touch panel 61, which is a display device. For example, the terminal output unit 60 outputs information by displaying the information on the touch panel 61. The method of outputting information is not limited to this example, and may be performed by outputting voice or the like from a speaker or the like. The terminal output unit 60 can output sound information acquired by recording the user's abdominal sounds to the information processing apparatus 100 under the control of the terminal processing unit 40.

The touch panel 61 functions as a display that displays information, and also accepts operations by the user. The touch panel 61 may be understood to be a part of the terminal acceptance unit 30. Note that a display device with only the function of displaying information may be used instead of the touch panel 61.

The terminal transmission unit 70 transmits information acquired by, for example, the terminal processing unit 40 via the network.

The sensor unit 80 includes the microphone 81 and an acceleration sensor 91. The sensor unit 80 records sounds using the microphone 81 and measures items to be measured, and outputs information such as the acquired audio data and the measurement values. The acquired information is accumulated in the terminal storage unit 10, for example. Here, examples of the measured values include a value indicating a change in acceleration obtained by the acceleration sensor, a value indicating a change in air pressure obtained by an air pressure sensor, and so on, but are not limited to these examples.

The microphone 81 is used to input the user's voice during a call, for example. In the present embodiment, the microphone 81 may be used to record the user's abdominal sounds. There is no limitation on the type, specific configuration, and so on of the microphone 81.

The acceleration sensor 91 is provided to detect the acceleration of the portable information terminal 6. The terminal processing unit 40 can detect the acceleration of the portable information terminal 6 using the acceleration sensor 91. That is to say, the terminal processing unit 40 can detect the orientation of the portable information terminal 6 when the portable information terminal 6 is substantially stationary.

Note that the sensor unit 80 may include, for example, a pulse sensor, an illuminance sensor, a camera, and a position information sensor that can specify a position using a GPS or the like. The sensor unit 80 may also be a timer that measures the passage of time. The portable information terminal 6 may be configured to function as an activity meter for acquiring information regarding the user's lifestyle habits, such as information regarding the user's activity level, such as calories burned and number of steps taken, the user's wake-up time, bedtime, and commute time, and so on. Note that information regarding the user's living environment, such as the area in which the user lives, the climate, the noise environment, etc., may be acquired based on the measurement values acquired by the sensor unit 80 and accumulated in the terminal storage unit 10.

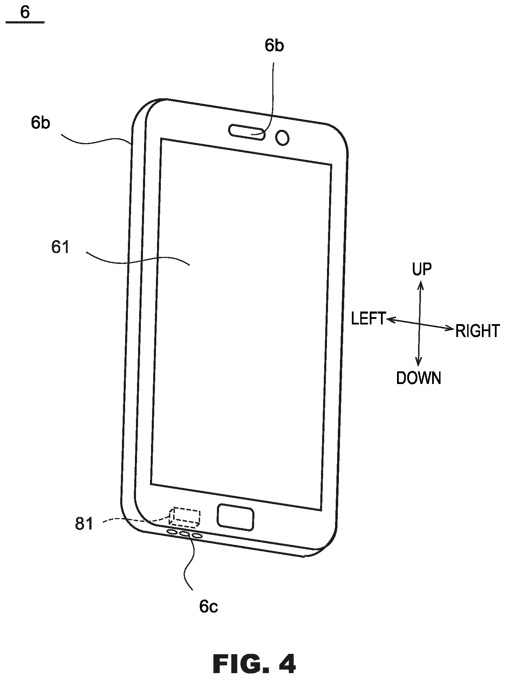

FIG. 4 is a perspective view showing the portable information terminal 6 according to the same.

In the present embodiment, the portable information terminal 6 has a housing 6b having a rectangular plate shape. One surface of the housing 6b is provided with the touch panel 61.

The microphone 81 is accommodated in the housing 6b. In the present embodiment, the microphone 81 is disposed near an end portion of the housing 6b. The portion of the housing 6b near the microphone 81 is a sound collecting portion 6c. The sound collecting portion 6c is, for example, an opening formed in the housing 6b so as to enable transmission of sound from the outside of the housing 6b to the microphone 81, but is not limited to this example. Note that, in addition to the microphone 81, a microphone built in another location may be provided. That is to say, the portable information terminal 6 may have two or more microphones. The recording of abdominal sounds is performed using one or more microphones, including at least the microphone 81.

In the present embodiment, the sound collecting portion 6c is disposed at an end portion of the housing 6b. It can be said that, when the surface on which the touch panel 61 is arranged is defined as the upper surface, the sound collecting portion 6c is disposed on a side edge portion of the housing 6b. The sound collecting portion 6c may also be said to be arranged on the side peripheral portion of the housing 6b.

More specifically, the sound collecting portion 6c is located at the bottom of the portable information terminal 6. The sound collecting portion 6c may also be said to be arranged at the lower edge of the housing 6b. The portable information terminal 6 is provided with an output port 6d for a speaker for calls at a top portion thereof. When the portable information terminal 6 is held close to the user's head for a call, the output port 6d is located at an upper portion close to the user's ear, and the sound collecting portion 6c is located at a lower portion close to the user's mouth. Note that, in the present embodiment, the term "lower" refers to the lower side of the display screen, contents, and the like that are displayed on the touch panel 61 when the portable information terminal 6 is used in a normal manner.

Note that a phone case (which may also be referred to as a protective cover) can be attached to the housing 6b of the portable information terminal 6 in order to protect the portable information terminal 6 from shocks, for example, when it is dropped. The phone case may be attached to the housing 6b for other purposes, such as decorative purposes.

In the present embodiment, abdominal sounds can be recorded using the portable information terminal 6, for example, in the manner described below.

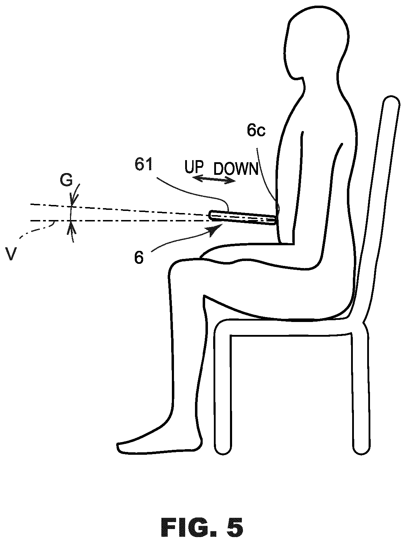

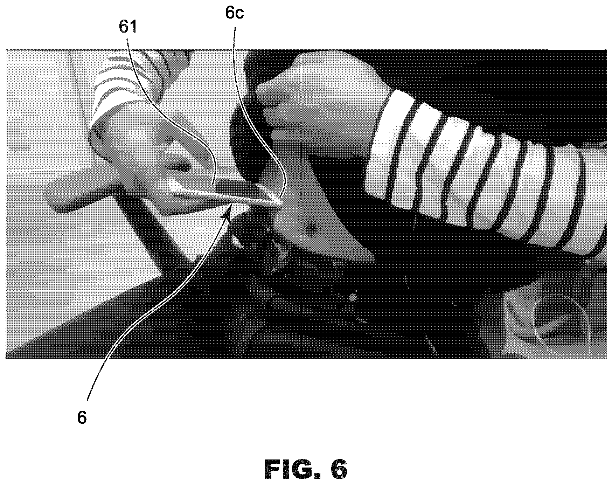

FIG. 5 is a diagram illustrating abdominal sound recording performed with the portable information terminal 6 according to the same. FIG. 6 is a diagram showing an example of a situation in which a user records abdominal sounds with the portable information terminal 6 according to the same.

As shown in the figures, in the present embodiment, the user's abdominal sounds are recorded with the sound collecting portion 6c of the housing 6b near the microphone 81 being brought into contact with the user's abdomen. In this case, the user is in a sitting position, sitting on a chair or the like, and is at rest. It is preferable that the sound collecting portion 6c is placed in direct contact with the skin of the abdomen. For adult users, the contact site is preferably located 9 cm to the left or right of the navel. In the present embodiment, the user starts sound recording while gently pressing the sound collecting portion 6c against the abdomen. The abdominal sound recording will stop after 60 seconds of recording.

Here, in the present embodiment, when the user is in a sitting position with the sound collecting portion 6c in contact with the abdomen, the portable information terminal 6 is preferably oriented such that the touch panel 61 faces upward. The user can check the display contents of the touch panel 61 before, during, or after sound recording. In this case, in order to acquire a higher-quality recording result, it is preferable that the touch panel 61, i.e., the portable information terminal 6, is held in a position such that it is approximately perpendicular to the plane that is in contact with the surface of the abdomen at the contact point. More specifically, it is preferable that the angle (which may also be referred to as a pressing angle G of the portable information terminal 6) between the line perpendicular to the plane that is in contact with the surface of the abdomen at the contact point (indicated by the symbol V in FIG. 5) and the touch panel 61 is substantially 90 degrees.

In the portable information terminal 6, the environment detection unit 41 and the sound recording processing unit 51 are provided as described above, and therefore the detection of the sound recording environment and processing corresponding to the result of the detection may be performed, for example, in the following manner.

First, the environment detection unit 41 may perform detection with respect to the environment in which the sound recording is performed, based on sound information acquired by the sound recording performed using the microphone 81. The sound information used at this time may be sound information recorded for the purpose of recording abdominal sounds, or a recording of sounds collected by the microphone 81 prior to recording abdominal sounds.

For example, the environment detection unit 41 may be configured to perform detection based on the noise level in the sound information acquired by the recording performed using the microphone 81, or the signal-to-noise ratio between abdominal sounds and noise. For example, the environment detection unit 41 may be configured to detect that the environment is an inappropriate environment (or is not an appropriate environment) when the noise level in the sound information is greater than or equal to a predetermined value or the signal-to-noise ratio is greater than or equal to a predetermined value. Note that the environment detection unit 41 may be configured to detect that the environment is an appropriate environment (or is not an inappropriate environment) when the noise level in the sound information is less than or equal to a predetermined value or the signal-to-noise ratio is less than or equal to a predetermined value.

The sound recording processing unit 51 may be configured such that when an inappropriate environment or an appropriate environment is detected, the sound recording processing unit 51 performs processing accordingly so that sound information is acquired in an appropriate environment.

Also, for example, the environment detection unit 41 may acquire information regarding whether or not a phone case is attached to the housing 6b, and detect the environment in which sound recording is performed based on the acquired information. For example, the environment detection unit 41 may acquire information regarding whether or not a phone case is attached to the housing 6b based on sound information. That is to say, when a phone case is attached to the housing 6b, the gut sounds tends to be highly detailed, but electromagnetic noise may occur in the sound information. When a phone case is not attached, gut sounds may sound a little muffled, but noise is less likely to occur. This is thought to be because when a phone case is attached, the housing 6b is likely to be in an electrically floating state depending on the structure of the housing 6b, etc., the phone case used, or a combination thereof. Taking advantage of this, in the present embodiment, the environment detection unit 41 can be configured to detect electromagnetic noise contained in the sound information, and to acquire information regarding whether or not a phone case is attached to the housing 6b based on the result of the detection of the electromagnetic noise. When the electromagnetic noise is relatively large, information indicating that a phone case is attached may be acquired. The environment detection unit 41 may be configured to detect that the environment is not an appropriate environment when it is determined that a phone case is attached to the housing 6b. Note that the fact that the environment detection unit 41 judges that a phone case is attached to the housing 6b may be expressed as the environment detection unit 41 detecting that the environment is an inappropriate environment, and the fact that the environment detection unit 41 judges that a phone case is not attached to the housing 6b may be expressed as the environment detection unit 41 detecting that the environment is an appropriate environment.

When the environment detection unit 41 judges that a predetermined condition regarding the quality of the sound information is satisfied, the environment detection unit 41 may acquire information regarding whether or not a phone case is attached to the housing 6b. The predetermined condition may be, for example, that the signal-to-noise ratio is less than or equal to a predetermined value, that the level of electromagnetic noise is greater than or equal to a predetermined value, or the like. The environment detection unit 41 may also perform the judgment based on, for example, sound information acquired before the recording of abdominal sounds and sound information during the sound recording. That is to say, for example, the environment detection unit 41 may judge that a phone case is attached if a condition that the volume of noise when the portable information terminal 6 is pressed against the abdomen before recording begins is greater than or equal to a predetermined value, and the volume of the gut sounds detected for the first time after recording begins is greater than or equal to a predetermined value is satisfied. Also, for example, if noise is detected continuously for a predetermined period of time (for example, 5 to 10 seconds) in a specified frequency band of 500 Hz or higher, the environment detection unit 41 may judge that this is electromagnetic noise and that a phone case is attached.

In addition, when it is judged that a phone case is attached in this way, the sound recording processing unit 51 may perform processing to stop recording that is being performed. In addition, the sound recording processing unit 51 may display a predetermined warning display on the touch panel 61. That is to say, when it is judged that a phone case is attached to the housing 6b, the sound recording processing unit 51 may display a warning display to provide a notification to the user of the portable information terminal 6 to prompt the user to remove the phone case. The warning display may also be information provide to ask the user whether or not a phone case is attached. The sound recording processing unit 51 may also again perform processing to acquire information regarding whether or not a phone case is attached to the housing 6b.

In addition, for example, the environment detection unit 41 may be configured to acquire information regarding the pressing force of the portable information terminal 6 against the abdomen based on the sound information, and detect the environment in which sound recording is performed based on the acquired information. The pressing force may be referred to as pressing pressure. In the present embodiment, the environment detection unit 41 may detect noise in the sound information, and acquire information regarding whether or not the pressing force is appropriate based on the result of the detection. That is to say, the volume of the noise in the sound information changes depending on the magnitude of the pressing force, and the environment detection unit 41 can acquire information regarding whether or not the pressing force is appropriate based on the volume of the noise. For example, the environment detection unit 41 can acquire information indicating that the pressing force is appropriate when the volume of the noise is less than a predetermined value. In addition, the volume of gut sounds in the sound information changes depending on the magnitude of the pressing force, and the the environment detection unit 41 can acquire information regarding whether or not the pressing force is appropriate based on the magnitude of the signal-to-noise ratio. For example, the environment detection unit 41 can acquire information indicating that the pressing force is appropriate when the magnitude of the signal-to-noise ratio is greater than a predetermined value.

For example, the environment detection unit 41 may be configured to detect that the environment is appropriate when information indicating that the pressing force is appropriate is acquired, and to detect that the environment is not appropriate when information indicating that the pressing force is not appropriate is acquired. Note that the fact that the environment detection unit 41 judges that the pressing force is appropriate may be expressed as detecting that the environment is appropriate, and the fact that the environment detection unit 41 judges that the pressing force is not appropriate may be expressed as detecting that the environment is inappropriate.

When it is judged that the pressing force is not appropriate, the sound recording processing unit 51 may perform processing to stop the recording that is being performed. In addition, in this case, the sound recording processing unit 51 may display a predetermined warning display on the touch panel 61. Here, the warning display may be information that guides the user to change the pressing force, or may be information that makes the user aware of the need to reduce the pressing force. This is because in a typical quiet environment, less pressing force is expected to result in less noise. In addition, the sound recording processing unit 51 may be configured to, when the pressing force is judged to be appropriate, output information to guide the user to maintain that state during sound recording.

Note that the environment detection unit 41 may detect a more appropriate pressing force so that a higher-quality recording result of abdominal sounds can be acquired. In this case, if the signal-to-noise ratio becomes the largest during a predetermined period (for example, from when the portable information terminal 6 is pressed against the abdomen until the present), it may be detected that the pressing force is optimal. In other words, the environment detection unit 41 may acquire information regarding the current pressing force based on past sound information and current sound information, and perform detection regarding the current pressing force based on the acquired information. When it is judged that the pressing force is optimal, or when it is judged that the pressing force is not optimal (the signal to noise ratio is greater at a point in time in the past), the sound recording processing unit 51 can output information to guide the user. As a result, the user can record abdominal sounds while adjusting the pressing force so as to maintain a state in which information indicating that the pressing force is optimal is output.

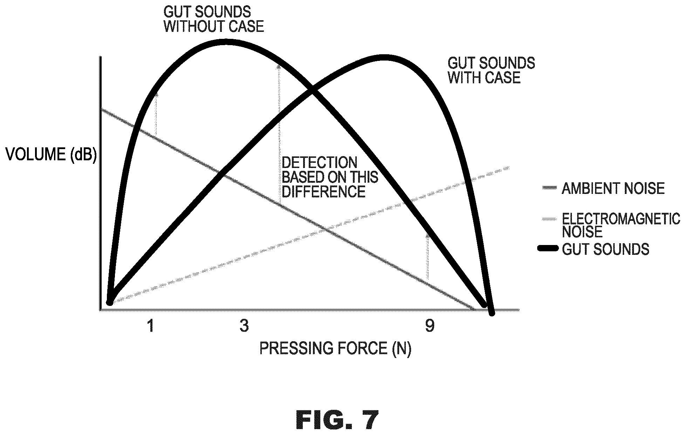

FIG. 7 is a graph showing an example of a relationship between the pressing force and the volume of noise in sound information.

In FIG. 7, the horizontal axis indicates the magnitude of the pressing force, and the vertical axis indicates the volume of gut sounds, noise, etc. In an environment where there is a certain amount of ambient noise, the volume of the ambient noise recorded as sound information decreases as the pressing force increases. Electromagnetic noise is generated when there is a phone case, and increases as the pressing force increases. When there is no case, the volume of the recorded gut sounds is maximum in a certain range where the pressing force is weak (approximately 2 N). On the other hand, when there is a phone case, the volume of the recorded gut sounds is maximum in a certain range where the pressing force is strong (approximately 9 N).

Since there is such a relationship between the pressing force and the volume of each sound, the environment detection unit 41 may perform detection regarding the optimal pressing force, for example, in the following manner. That is to say, when there is no phone case, it can be judged that the pressing force that maximizes the signal-to-noise ratio between ambient noise and gut sounds is optimal. For example, in the case of the relationship shown in FIG. 7, it can be seen that approximately 2N to 3N is optimal. On the other hand, when there is a phone case, it can be judged that the pressing force that maximizes the signal-to-noise ratio between ambient noise or electromagnetic noise and gut sounds is optimal. For example, in the case of the relationship shown in FIG. 7, it can be seen that approximately 7N to 9N is optimal. Note that when there is a phone case, the pressing force may be judged to be too strong if the volume of the noise generated when the portable information terminal 6 is pressed against the abdomen is greater than or equal to a predetermined value.

In addition, for example, the environment detection unit 41 may detect the environment in which recording is performed based on information regarding the orientation of the recording apparatus. In the present embodiment, when the user is in a sitting position with the sound collecting portion 6c in contact with the abdomen, the environment detection unit 41 performs detection based on information regarding whether or not the angle (pressing angle G) between the touch panel 61 and a line perpendicular to the plane that is in contact with the surface of the abdomen at the contact point is within a predetermined angle range. The pressing angle G can be detected with the acceleration sensor 91, for example.

In the present embodiment, the environment detection unit 41 performs detection by acquiring information regarding whether or not the pressing angle G is within a range of approximately 30 degrees, for example. The microphone 81 is configured to have high sensitivity to sounds arriving from below, and if the pressing angle G is within the predetermined angle range, the environment is an appropriate environment in which gut sounds included in abdominal sounds can be recorded at a relatively loud volume.

Note that the angle range of the pressing angle G is not limited to this example. For example, the pressing angle G may be within the range of 20 degrees. The portable information terminal 6 is pressed against the abdomen with the touch panel 61 facing upward, and the pressing angle G may be said to be the pitch angle of the portable information terminal 6. It may be said that the pressing angle G is an angle around an axis parallel to the left-right direction of the touch panel 61. The pressing angle may also be said to be the pitch angle of the portable information terminal 6 relative to the upright position thereof. In this case, the environment detection unit 41 may be configured to detect that the environment is an appropriate environment when the pressing angle is within the angle range of 70 degrees to 120 degrees, for example.

In this way, the sound recording processing unit 51 may be configured to output, when it is detected that the environment is an appropriate environment with respect to the orientation of the portable information terminal 6, information to guide the user to maintain that state during sound recording.

Note that the environment detection unit 41 may be configured to detect whether or not the portable information terminal 6 is moving or stopped using the acceleration sensor 91, and detect that the environment is an appropriate environment when it is detected that the portable information terminal 6 is stopped. As a result, it is possible to record abdominal sounds in a stable state, and accordingly acquire a high-quality recording result of abdominal sounds.

The environment detection unit 41 may acquire device identification information from the device identification information storage unit 13 and perform detection based on the acquired identifier. For example, the environment detection unit 41 may perform detection regarding the presence or absence of a phone case as described above for certain models in which the presence or absence of a phone case has a large effect on electromagnetic noise, but may not detect the presence or absence of a phone case for other models. In this way, it becomes possible to record high-quality abdominal sounds for each model of portable information terminal 6.

Next, examples of operations of the portable information terminal 6 and examples of operations of the information processing apparatus 100 performed when a user uses the information processing system 1 according to the present embodiment will be described. In the present embodiment, a user can use the information processing system 1, for example, by accessing the information processing apparatus 100 and receiving information transmitted from the information processing apparatus 100 using the portable information terminal 6, and by running a predetermined application on the portable information terminal 6. Note that the predetermined application may be, for example, a dedicated application that runs using information transmitted from the information processing apparatus 100, or a web browser or the like that displays web applications provided in the information processing apparatus 100 so that the user can use the applications.

In the present embodiment, the information processing system 1 is typically used in the following manner. That is to say, the user uses the portable information terminal 6 to record their own abdominal sounds. The sound information is transmitted from the portable information terminal 6 to the information processing apparatus 100, and the gut score is acquired in the information processing apparatus 100. The information processing apparatus 100 transmits (outputs) the acquired gut score to the portable information terminal 6. The portable information terminal 6 receives the gut score and displays information including the gut score on the display device via the terminal output unit 60. As a result, the user can confirm information regarding their own intestines as a gut score. When such operations of the information processing system 1 are performed, the portable information terminal 6 and the information processing apparatus 100 perform various operations in the following manner, for example. These operations are performed by the terminal processing unit 40 and the processing unit 140 performing control operations or the like using each unit.

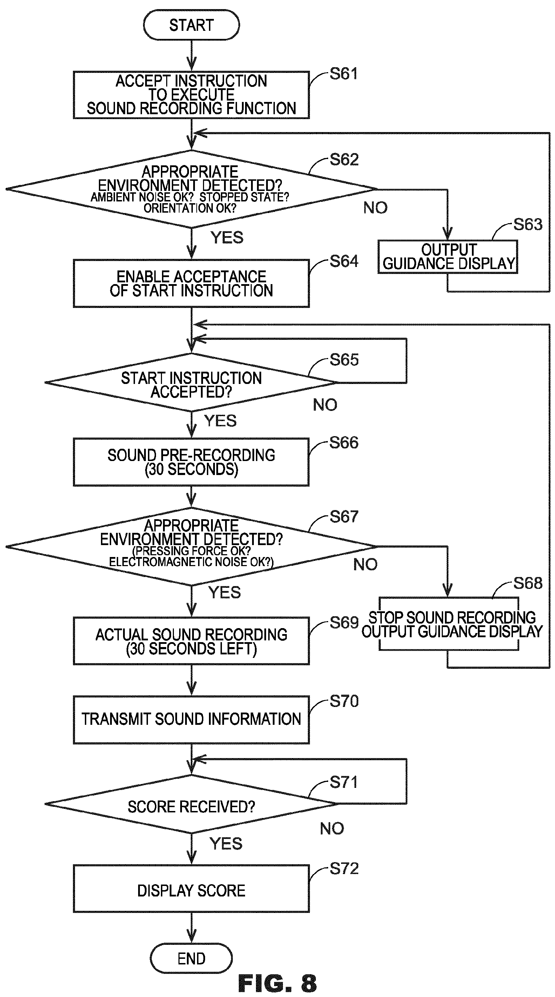

FIG. 8 is a flowchart showing examples of operations of the information processing apparatus 100 according to the same.

(Step S61) The terminal processing unit 40 receives an instruction to execute the sound recording function of recording abdominal sounds. For example, when an instruction to start a predetermined application or an operation to select a predetermined operation mode is performed, the terminal processing unit 40 accepts an instruction to execute the sound recording function.

To perform sound recording, the user takes a sitting position and presses the sound collecting portion 6c of the portable information terminal 6 against their abdomen.

(Step S62) The terminal processing unit 40 judges whether or not it is detected that the environment is an appropriate environment. Here, for example, it is detected that the environment is an appropriate environment when all of the following conditions are satisfied: the volume of the ambient noise is smaller than a predetermined value; the portable information terminal 6 is in a substantially stopped state; and the orientation of the portable information terminal 6 is in a predetermined orientation (the pressing angle G is within a predetermined angle range). If it is detected that the environment is an appropriate environment, processing proceeds to step S64, and otherwise processing proceeds to step S63.

(Step S63) The terminal processing unit 40 outputs a guidance display indicating that the environment is not an appropriate environment. For example, the information is displayed on the touch panel 61. Thereafter, processing returns to step S62.

(Step S64) The terminal processing unit 40 controls the portable information terminal 6 so that the portable information terminal 6 can accept a start instruction from the user. For example, an operation button for starting recording of abdominal sounds (for starting recording of abdominal sounds as sound information) is enabled on the operation interface displayed on the touch panel 61. For example, the display state may be changed, such as by making the button appear or canceling the grayed-out state, or the behavior when the operation button is operated may be changed.

(Step S65) The terminal processing unit 40 judges whether or not a start instruction has been accepted from the user. The terminal processing unit 40 waits until a start instruction is accepted, and processing proceeds to step S66 when the start instruction is accepted.

(Step S66) The terminal processing unit 40 performs sound pre-recording. The sound pre-recording is performed for a predetermined period of time (for example, 30 seconds). The sound information acquired by sound pre-recording is included in the sound information ultimately used to acquire the gut score, but is not required to be used to obtain the gut score.

(Step S67) The terminal processing unit 40 judges whether or not it is detected that the environment is an appropriate environment based on the sound information acquired by the sound pre-recording. Here, for example, it is detected that the environment is an appropriate environment when all of the following conditions are satisfied: the pressing force is appropriate; and the magnitude of electromagnetic noise is less than or equal to a predetermined value. If it is detected that the environment is an appropriate environment, processing proceeds to step S69, and otherwise processing proceeds to step S68.

(Step S68) The terminal processing unit 40 interrupts the sound recording and outputs a guidance display indicating that the environment is not an appropriate environment. For example, the information is displayed on the touch panel 61. Thereafter, processing returns to step S65. note that processing may return to other processing or the series of processing may be terminated.

(Step S69) The terminal processing unit 40 performs actual sound recording. The actual sound recording is performed for a predetermined period of time (for example, 30 seconds). As a result, a recording result of the abdominal sounds during the period of time required to acquire a score is acquired together with the pre-recording. It is also possible to only use the actual recording as the sound information used to acquire the gut score.

While sound pre-recording and actual sound recording are being performed as described above, the terminal processing unit 40 may be configured to output a display related to the sound recording status on the touch panel 61 so that the user can visually check it. The display related to the sound recording status may be, for example, a display showing the progress status (for example, a display showing the remaining time, a progress bar, or the like). The sound recording status display can be a waveform or the like that shows the contents of the recorded sound information. Providing such a display helps the user maintain the stopped state necessary to record high-quality abdominal sounds.

(Step S70) The terminal processing unit 40 transmits the sound information to the information processing apparatus 100. Note that if the sound information cannot be transmitted properly due to a communication problem or the like, a display may be displayed suggesting to the user to move to another location or change the communication means.

As a result, when the sound information is transmitted to the information processing apparatus 100, the sound information is used in the information processing apparatus 100 to acquire a gut score, and information regarding the acquire gut score or the like is transmitted from the portable information terminal 6 to the information processing apparatus 100.

(Step S71) The terminal processing unit 40 judges whether or not information regarding the score transmitted from the information processing apparatus 100 has been received. The terminal processing unit 40 waits until the information is received, and processing proceeds to step S72 when the start instruction is received.

(Step S72) The terminal processing unit 40 uses the received information regarding the score to output a display related to the gut score or the like on the touch panel 61. As a result, the user can know their own gut score or related information. Thereafter, the series of processing is terminated.

Note that in the processing performed to detect an appropriate environment described above, the conditions for detecting that the environment is an appropriate environment may be a combination of multiple conditions as described above, or may be a condition related to any one of the aspects. For example, in step S62, it may be detected that the environment is an appropriate environment based on the result of the judgment only regarding the volume of the ambient noise.

In addition, based on the results of the sound pre-recording or the actual sound recording, if there is an abnormality such as the abdominal sounds do not contain gut sounds or there are more than a certain volume of sounds judged to be gut sounds, the user may be notified of this abnormality or be suggested to perform the sound recording again. The presence or absence of such an abnormality may be judged within the portable information terminal 6 based on the sound information acquired in step S66 or step S69, or the sound information may be transmitted to the information processing apparatus 100 and the result of the judgment performed by the information processing apparatus 100 may be fed back to the portable information terminal 6.

Since the portable information terminal 6 is configured to perform such operations, the sound recording method for recording the user's abdominal sounds using the portable information terminal 6 can be said to include the following steps. That is to say, first, the user brings the sound collecting portion 6c into contact with the user's abdomen in a state where the user is in a sitting position (first step). Next, the terminal processing unit 40 causes the environment detection unit 41 to perform detection with respect to an appropriate environment with the sound collecting portion 6c in contact with the abdomen (second step). In other words, the user causes the portable information terminal 6 to perform detection with respect to the appropriate environment with the sound collecting portion 6c in contact with the abdomen. Thereafter, the terminal processing unit 40 causes the sound recording processing unit 51 to perform processing related to sound recording based on the result of the detection by the environment detection unit 41 (third step). In other words, the user uses the portable information terminal 6 to enable processing related to sound recording according to the result of the detection. By using such a recording method, a high-quality recording result can be obtained.

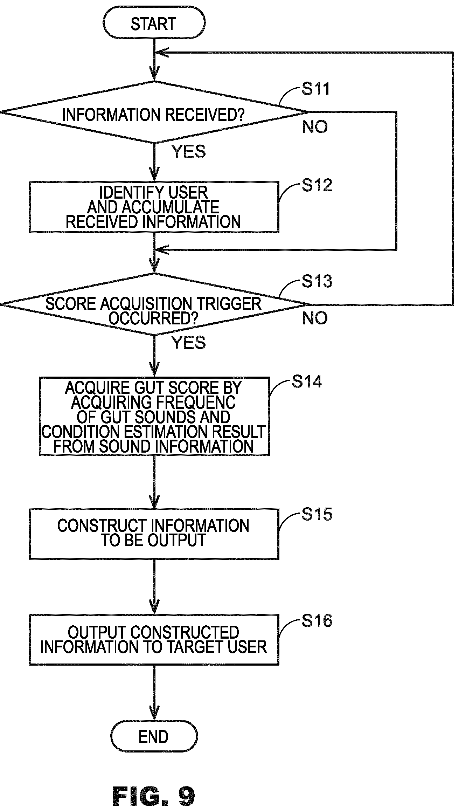

FIG. 9 is a flowchart showing examples of operations of the information processing apparatus 100 according to the same.

(Step S11) The processing unit 140 judges whether or not the reception unit 120 has received information transmitted from the portable information terminal 6 or the like. If it is judged that information has been received, processing proceeds to step S12, and otherwise processing proceeds to step S13.

(Step S12) The processing unit 140 identifies the user based on the received information, and accumulates the received information in the user information storage unit 115 in association with the user identifier. For example, when lifestyle information, sound information, device identification information, or the like is transmitted from the portable information terminal 6 in association with the user identifier, the processing unit 140 accumulates the received information in the user information storage unit 115 in association with the user identifier.

(Step S13) The processing unit 140 judges whether or not a trigger for acquiring a gut score has occurred. In other words, the processing unit 140 judges whether or not the conditions for starting to acquire a gut score are satisfied. If it is judged that a trigger has occurred, processing proceeds to step S14. Otherwise, processing returns to step S11.

Note that, for example, the above trigger can be an instruction from the user via the portable information terminal 6 to acquire a gut score (the transmission of predetermined information corresponding to the instruction), but the trigger is not limited to this example. For example, the trigger can be the satisfaction of various conditions, such as reaching a predetermined point in time, receiving new sound information or other lifestyle information, and so on.

(Step S14) The processing unit 140 performs score acquisition processing, using the gut score acquisition unit 149. Score acquisition processing will be described later. A gut score and each element score are acquired and accumulated in the user information storage unit 115 through score acquisition processing.

(Step S15) The processing unit 140 causes the gut score output unit 161 to construct information to be output, using the acquired gut score and so on. In the present embodiment, information for showing the gut score and information for showing the element scores as a radar chart are constructed.

(Step S16) The processing unit 140 causes the gut score output unit 161 to output the constructed information to the target user. That is to say, the processing unit 140 outputs, via the transmission unit 170, the information constructed with respect to the gut score and the element scores to the portable information terminal 6 used by the user for whom the score is calculated. As a result, it is possible to display the gut score and the element scores on the portable information terminal 6 that has received the information.

Next, specific examples of the sound recording of abdominal sounds according to the present embodiment will be described while showing screen transitions on the portable information terminal 6 used by the user.

In the following specific examples, it is assumed that the information processing system 1 provides a health support application to assist the user in leading a healthy life. The health support application accepts input operations related to the user's lifestyle information and provides the user with information useful for maintaining health. Th health support application is realized by the user executing a predetermined application on the portable information terminal 6, and the portable information terminal 6 and the information processing apparatus 100 performing communication with each other. The example screens of the health support application described below are displayed by the terminal output unit 60 under the control of the terminal processing unit 40.

While the health support application is running, the user can record their own abdominal sounds to acquire a gut score, by performing a predetermined operation.

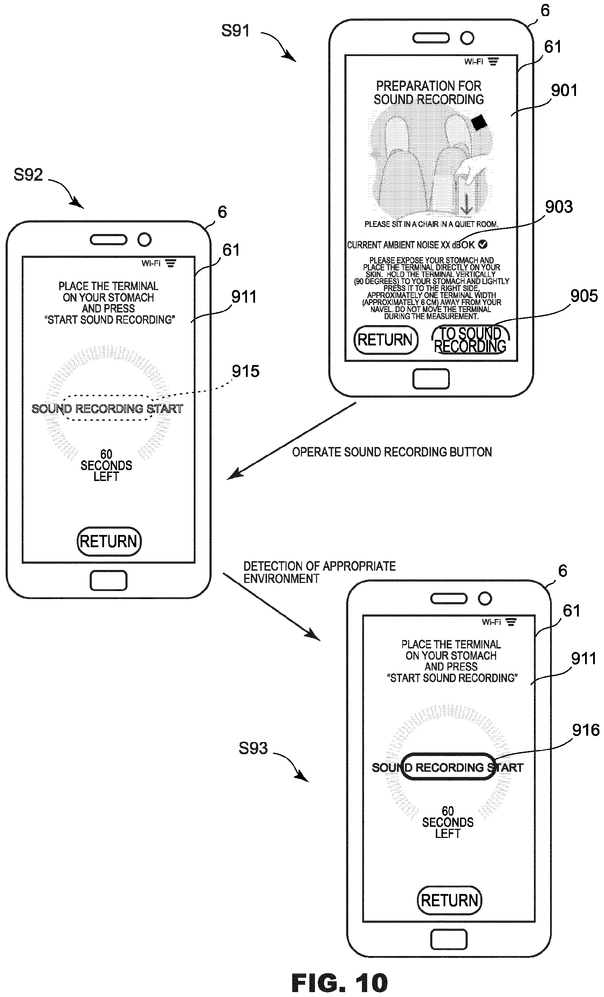

FIG. 10 is a first diagram showing specific examples of screen transitions of the portable information terminal 6 according to the same.

In FIG. 10, in step S91, a sound recording preparation screen 901 for performing sound recording using the health support application is displayed. The sound recording preparation screen 901 includes, for example, a display guiding the user to sit down and press the portable information terminal 6 against their abdomen in order to record sounds. The user makes the necessary preparations according to the displayed contents of the recording preparation screen 901, and the user can cause the screen to transition to the next screen to proceed to the sound recording by operating a sound recording button 905.

In the example shown in the figure, the sound recording preparation screen 901 includes an environment information display 903 that shows the result of measuring the ambient noise and the result of detecting that the environment is an appropriate environment. As a result, the user can easily know information regarding ambient noise, such as whether or not the environment is an appropriate environment and the volume of the ambient noise. The display mode of the environment information display 903 may be changed depending on whether the environment is detected as an appropriate environment or not. Also, the configuration may be such that the operation on the sound recording button 905 can be accepted only when the environment is detected as an appropriate environment.

When the sound recording button 905 is operated, the screen transitions to a sound recording start instruction screen 911 (step S92). On the sound recording start instruction screen 911, initially, a sound recording start button 915 is displayed in an inactive state (not ready to accept an operation). Note that the sound recording start button 915 does not necessarily have to be displayed in the first place. In the case where the sound recording start instruction screen 911 is displayed, if it is detected that the environment is an appropriate environment as described above with respect to the orientation and movement of the portable information terminal 6, a sound recording start button 916 that is ready to accept an operation is displayed (step S93).

As a result, the user can start recording abdominal sounds by to operating the recording start button 916. The recording of abdominal sounds starts in the state where the environment has been detected as an appropriate environment, and therefore it is possible to reliably acquire a high-quality recording result of abdominal sounds.

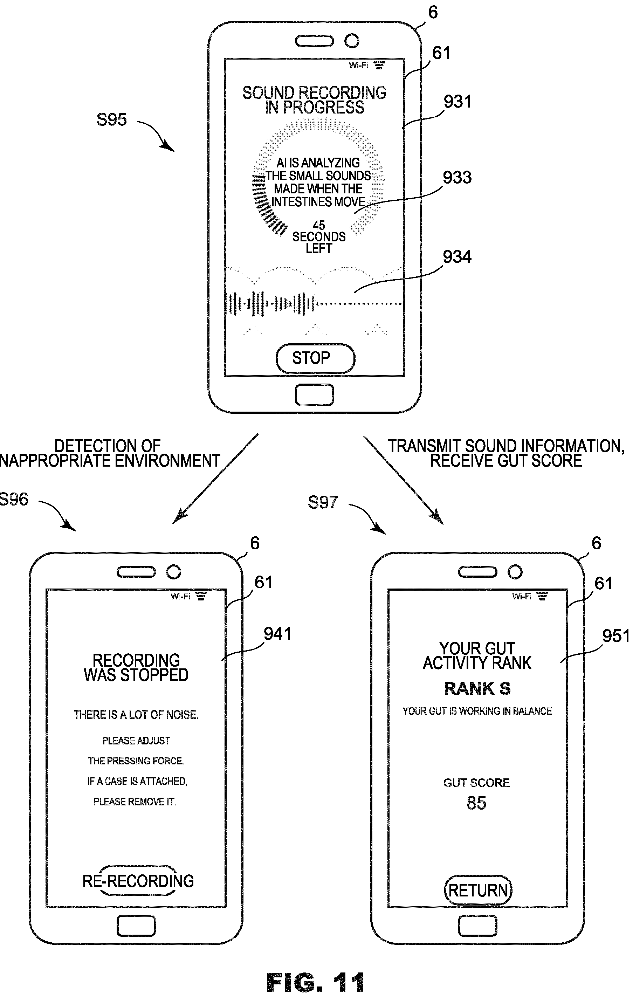

FIG. 11 is a second diagram showing specific examples of screen transitions of the portable information terminal 6 according to the same.

In FIG. 11, in step S95, a recording-in-progress screen 931 is displayed, indicating that abdominal sounds are being recorded. The recording-in-progress screen 931 shows a recording progress display 933 and a recording waveform display 934 as displays related to the sound recording status. The recording progress display 933 may be, for example, a remaining time indicator or a progress bar indicating the degree of progress, but is not limited to this example. The recording waveform display 934 displays a waveform indicating the contents of the recorded sound information and allows the user to have a realistic sense that sound recording is in progress.

In the example shown in the figure, the recording-in-progress screen 931 includes such display of the sound recording status, and therefore it is possible to attract the user's attention during the sound recording and prevent the user from making unnecessary movements that are one of the factors that may reduce the quality of the sound recording. In addition, the user can check the progress, and therefore the user can wait for the sound recording to be completed without worrying.

Here, if it is detected during sound recording that the environment is not an appropriate environment, the screen transitions to an error screen 941 (step S96). For example, if it is detected that there is a high level of noise or that the pressing force is not appropriate based on the sound information indicating the result of the pre-recording, the screen can transition to the error screen 941. The error screen 941 may include information indicating that the sound recording has been stopped or information regarding the cause and a solution to the problem. The user can check the contents of the error screen 941 and then try to record the sounds again.

On the other hand, if the sound recording is performed normally, the sound information is transmitted to the information processing apparatus 100, and the gut score is transmitted from the information processing apparatus 100 to the portable information terminal 6. When the portable information terminal 6 receives the gut score, the screen transitions to a result display screen 951 (step S97). The result display screen 951 displays, for example, the acquired gut score and information regarding the score (for example, information indicating a rank classified according to the degree of gut activity, etc.). The user can check the contents of the result display screen 951, recognize an objective evaluation of the condition of their own intestines, and use this information in their daily life.

As described above, in the present embodiment, the processing related to sound recording is performed using the portable information terminal 6 based on the result of the detection that the environment is an appropriate environment, and therefore a high-quality recording result of abdominal sounds can be acquired. When the environment is not an appropriate environment, a corresponding display is displayed on the touch panel 61, thereby urging the user to record sounds in a more appropriate environment. Note that a high-quality recording result specifically means, but is not limited to, a high signal-to-noise ratio, and can mean, for example, a high level of abdominal sounds, a low level of noise, or other conditions in which abdominal sounds (especially gut sounds) can be more clearly heard.

The information processing apparatus 100 uses high-quality sound information to acquire a gut score related to the user's gut condition, and outputs the acquired gut score, and therefore the information processing apparatus 100 can output a gut score that accurately represents the user's gut condition. Since such a gut score is output, the user can easily know objective information regarding the condition of their own intestines, which can be easily grasped.

It is preferable that the above storage unit 110 and the terminal storage unit 10 are realized using a non-volatile recording medium, but they can be realized using a volatile recording medium. Information or the like acquired by the apparatuses is stored in the storage units, but there is no limitation on the process in which information or the like is stored therein. For example, information or the like may be stored via a recording medium, or information or the like transmitted via a communication line or the like may be stored, or information or the like input via an input device may be stored.

The above processing unit 140 and the terminal processing unit 40 are typically realized using an MPU, a memory, or the like. The processing procedures performed by the processing unit 140 and the terminal processing unit 40 are typically realized using software, and the software is recorded on a recording medium such as a ROM. However, such processing procedures may be realized using hardware (a dedicated circuit).

In addition, the input means that can be used to input information that can be accepted by the acceptance unit 130 and the terminal acceptance unit 30 may be any means, such as a numeric keypad, a keyboard, a mouse, or a menu screen. The acceptance unit 130 and the terminal acceptance unit 30 can be realized by a device driver for an input means such as a numeric keypad or a keyboard, or control software for a menu screen.

In addition, the reception unit 120 and the terminal reception unit 20 are typically realized using a wireless or wired communication means, but may be realized using a broadcast reception means.

In addition, the transmission unit 170 and the terminal transmission unit 70 are typically realized using a wireless or wired communication means, but may be realized using a broadcast means.

Note that the information processing apparatus 100 may be constituted by a single server, or may be constituted by multiple servers operating in cooperation with each other, or may be a computer or the like built into other devices. Note that the server may be a so-called cloud server, an ASP server, or the like, and of course the type thereof is not limited.

In addition, some or all of the units provided in the information processing apparatus 100 described above may be provided in the portable information terminal 6. That is to say, the portable information terminal 6 may have a part or all of the configuration for realizing the functions related to acquiring and outputting the gut score, such as those of the information processing apparatus 100 described above. For example, the portable information terminal 6 may be configured to be able to acquire the gut score, using sound information recorded by the portable information terminal 6. On the other hand, some of the units provided in the portable information terminal 6 described above may be provided in the information processing apparatus 100. That is to say, the information processing apparatus 100 may include a part of the configuration for detecting whether or not the environment is an appropriate environment and for realizing processing related to recording corresponding to the detection, as the portable information terminal 6 described above includes. For example, the information processing apparatus 100 may be configured to perform processing such as specifying noise using sound information recorded by the portable information terminal 6 or to transmit an instruction to start recording or stop recording, for example, based on the result of the detection of an appropriate environment to the portable information terminal 6.

The processing in the present embodiment may be realized using software. This software may be distributed through software downloading or the like. Also, this software may be recorded on a recording medium such as a CD-ROM and distributed.

Note that the software that realizes the information processing apparatus 100 in the present embodiment is the program described below. That is to say, this program is a program that enables a computer to function as: a sound information acquisition unit that acquires sound information regarding a user's abdominal sounds, which are sounds emitted from the user's abdomen; a gut score acquisition unit that acquires a gut score related to the user's gut condition, using input information containing the sound information acquired by the sound information acquisition unit and learning information prepared in advance; and an output unit that outputs the gut score acquired by the gut score acquisition unit.

The software that realizes the portable information terminal 6 in the present embodiment, which is a sound recording apparatus, is the program described below. That is to say, this program is a program to be executed by a computer of a sound recording apparatus that has a microphone accommodated in a housing thereof and is used to record a user's abdominal sounds in a state where a sound collecting portion of the housing near the microphone is in contact with the user's abdomen, the program enabling the computer of the recording apparatus to function as: an environment detection unit that detects at least one of an inappropriate environment that is inappropriate for recording the abdominal sounds and an appropriate environment that is appropriate for recording the abdominal sounds; and a sound recording processing unit that performs processing related to sound recording based on a result of detection by the environment detection unit.

### Others

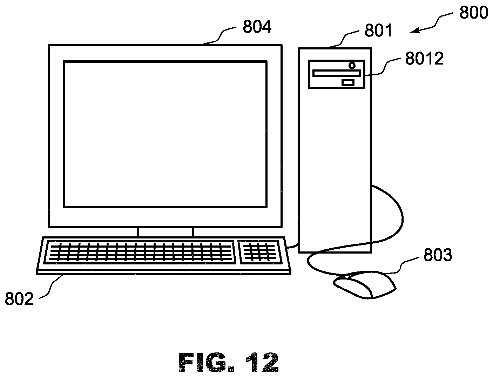

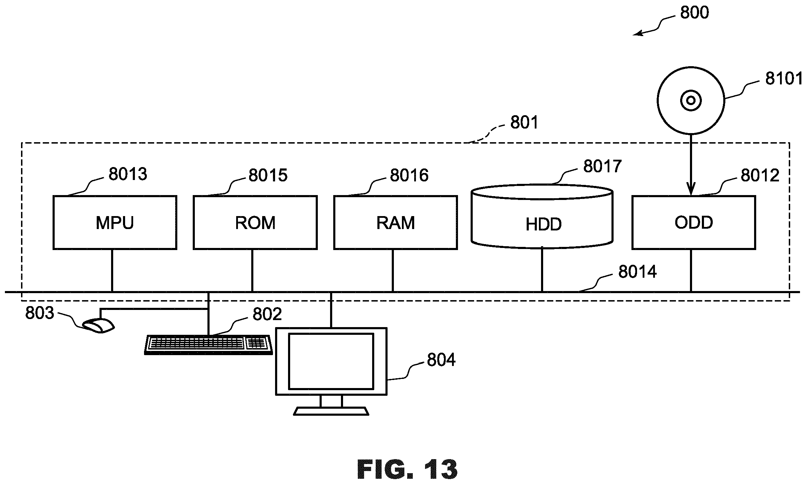

FIG. 12 is an overview diagram for a computer system 800 in the above embodiment. FIG. 13 is a block diagram for the computer system 800.

These figures show an example of the configuration of a computer that executes the program described in this specification to realize the information processing apparatus 100 and the like according to the above-described embodiments. The above-described embodiments can be realized using computer hardware and a computer program executed thereon.

The computer system 800 includes a computer 801 that includes an optical disc drive, a keyboard 802, a mouse 803, and a monitor 804.

The computer 801 includes, in addition to the optical disc drive (ODD) 8012, an MPU 8013, a bus 8014 that is connected to the optical disc drive 8012 and so on, a ROM 8015 for storing programs such as a boot-up program, a RAM 8016 that is connected to the MPU 8013 and is used to temporarily store application program instructions and provide a temporary storage space, and a hard disk (HDD) 8017 for storing application programs, system programs, and data. Here, although not shown in the figure, the computer 801 may further include a network card that provides connection to a LAN.

The program that enables the computer system 800 to perform the functions of the information processing apparatus and so on according to the above-described embodiments may be stored in the optical disc 8101, inserted into the optical disc drive 8012, and furthermore transferred to the hard disk 8017. Alternatively, the program may be transmitted to the computer 801 via a network (not shown) and stored on the hard disk 8017. The program is loaded into the RAM 8016 when the program is to be executed. The program may be loaded from the optical disc 8101 or directly from the network.

The program does not necessarily have to include an operating system (OS), a third-party program, or the like that enables the computer 801 to perform the functions of the information processing apparatus and so on according to the above-described embodiments. The program need only contain the part of the instruction that calls an appropriate function (module) in a controlled manner to achieve a desired result. How the computer system 800 works is well known and the detailed descriptions thereof will be omitted.

In the above-described program, the step of transmitting information, the step of receiving information, and so on do not include processing performed by hardware, for example, processing performed by a modem or an interface card in the step of transmitting (processing that can only be performed by hardware).

There may be a single or multiple computers executing the above-described program. That is to say, centralized processing or distributed processing may be performed.

In the above-described embodiments, two or more constituent elements that are present in one apparatus may be physically realized using one medium.

Also, in the above-described embodiments, each kind of processing (each function) may be realized as centralized processing that is performed by a single apparatus (system), or distributed processing that is performed by multiple apparatuses (in this case, the entire system constituted by multiple apparatuses performing distributed processing can be considered as a single "apparatus").

In addition, in the above-described embodiments, the transfer of information between constituent elements may be performed, for example, by one constituent element outputting information and the other constituent element receiving information if the two constituent elements transferring the information are physically different, or, by shifting from a processing phase corresponding to one constituent element to a processing phase corresponding to the other constituent element if the two constituent elements transferring the information are physically the same.

In addition, in the above-described embodiments, information related to the processing performed by each constituent element, for example information accepted, acquired, selected, generated, transmitted, or received by each constituent element, and information such as threshold values, formulas, addresses, etc. used by each constituent element in processing, may be stored temporarily or for a long period of time on a recording medium (not shown), even if not explicitly stated in the above description. In addition, the accumulation of information in a recording medium (not shown) may be performed by each constituent element or an accumulation unit (not shown). In addition, reading of information from the recording medium (not shown) may be performed by each constituent element or a reading unit (not shown).

In addition, in the above-described embodiments, if information used by each constituent element, such as threshold values, addresses, various setting values, etc. used by each constituent element in processing may be changed by the user, the user may or may not be able to change such information as appropriate, even if it is not explicitly stated in the above description. If these pieces of information can be changed by the user, the change may be realized, for example, by an acceptance unit (not shown) that accepts a change instruction from the user, and a change unit (not shown) that changes the information in accordance with the change instruction. The acceptance of the change instruction by the acceptance unit (not shown) may be, for example, acceptance from an input device, reception of information transmitted via a communication network, or acceptance of information read out from a predetermined recording medium.

The present invention is not limited to the above-described embodiments, and various modifications are possible, which are also included within the scope of the present invention.

Some of the component elements and functions of the above-described embodiments may be omitted.

### Industrial Applicability

As described above, the sound recording apparatus according to the present invention has the effect of being able to acquire a high-quality recording result of abdominal sounds, and is useful as a sound recording apparatus or the like.

### List of Reference Numerals

1 Information Processing System
6 Portable Information Terminal
10 Terminal Storage Unit
11 Sound Information Storage Unit
13 Device Identification Information Storage Unit
15 Lifestyle Information Storage Unit
20 Terminal Reception Unit
30 Terminal Acceptance Unit
40 Terminal Processing Unit
41 Environment Detection Unit
51 Sound Recording Processing Unit
60 Terminal Output Unit
61 Touch Panel
70 Terminal Transmission Unit
80 Sensor Unit
81 Microphone
91 Acceleration Sensor
100 Information Processing Apparatus
110 Storage Unit
111 Learning Information Storage Unit
115 User Information Storage Unit
120 Reception Unit
130 Acceptance Unit
140 Processing Unit
141 Sound Information Acquisition Unit
149 Gut score Acquisition Unit
161 Gut score Output Unit
170 Transmission Unit

## Claims

1. A sound recording apparatus that has a microphone accommodated in a housing thereof and is used to record a user's abdominal sounds in a state where a sound collecting portion of the housing near the microphone is in contact with the user's abdomen, the sound recording apparatus comprising:
an environment detection unit that detects at least one of an inappropriate environment that is inappropriate for recording the abdominal sounds and an appropriate environment that is appropriate for recording the abdominal sounds; and
a sound recording processing unit that performs processing related to the sound recording based on a result of detection by the environment detection unit.

2. The sound recording apparatus according to claim 1,
wherein the environment detection unit acquires information regarding a pressing force of the sound recording apparatus against the abdomen based on sound information acquired through sound recording performed using the microphone, and performs the detection based on the acquired information.

3. The sound recording apparatus according to claim 2,
wherein the environment detection unit detects noise in the sound information, and acquires information regarding the pressing force based on a result of the detection.

4. The sound recording apparatus according to claim 2,
wherein the environment detection unit acquires information regarding a current pressing force based on past sound information and current sound information, and performs the detection regarding the current pressing force based on the acquired information.

5. The sound recording apparatus according to claim 1,
wherein the environment detection unit performs the detection based on a sound-to-noise ratio between abdominal sounds and noise in sound information acquired through sound recording performed using the microphone.

6. The sound recording apparatus according to claim 1,
wherein the sound recording apparatus is a smartphone in which a phone case is attachable to the housing, and
the environment detection unit acquires information regarding whether or not a phone case is attached to the housing, and performs the detection based on the acquired information.

7. The sound recording apparatus according to claim 6,
wherein the environment detection unit detects electromagnetic noise contained in sound information acquired through sound recording performed using the microphone, and acquires information regarding whether or not a phone case is attached to the housing based on a result of detection of the electromagnetic noise.

8. The sound recording apparatus according to claim 6,
wherein the environment detection unit acquires information regarding whether or not a phone case is attached to the housing when a predetermined condition regarding quality of sound information acquired through sound recording performed using the microphone is satisfied.

9. The sound recording apparatus according to claim 6,
wherein when a fact that a phone case is attached to the housing is detected, the sound recording processing unit provides a notification to the user of the sound recording apparatus to prompt the user to remove the phone case.

10. The sound recording apparatus according to claim 1,
wherein the environment detection unit performs the detection based on information regarding an orientation of the sound recording apparatus.

11. The sound recording apparatus according to claim 10,
wherein the sound recording apparatus is a smartphone whose housing has a plate shape and a touch panel is disposed on one surface thereof,
the sound collecting portion is located at an end portion of the housing, and
the environment detection unit performs the detection when the user is in a sitting position and the sound collecting portion is in contact with the abdomen, based on information regarding whether an angle between the touch panel and a line perpendicular to a plane that is in contact with a surface of the abdomen at a contact site is within a predetermined angle range.

12. The sound recording apparatus according to claim 1,
wherein the sound recording processing unit is configured to output predetermined guidance information from the sound recording apparatus when a fact that the environment is the inappropriate environment or a fact that the environment is not the appropriate environment is detected.

13. The sound recording apparatus according to claim 1,
wherein the sound recording processing unit is configured to perform predetermined processing to carry out sound recording using the microphone or to use sound information acquired through sound recording performed using the microphone when a fact that the environment is the appropriate environment or a fact that the environment is not the inappropriate environment is detected.

14. The sound recording apparatus according to claim 13,
wherein the predetermined processing is to transmit the sound information to an external apparatus.

15. An information processing system comprising: the sound recording apparatus according to claim 1; and an information processing apparatus that can communicate with the sound recording apparatus,
wherein the sound recording apparatus includes a terminal output unit that outputs sound information acquired by recording a user's abdominal sounds using the microphone, and
the information processing apparatus includes:
a sound information acquisition unit that acquires the sound information;
a gut score acquisition unit that acquires a gut score related to the user's gut condition, using input information containing the sound information acquired by the sound information acquisition unit and learning information prepared in advance; and
a gut score output unit that outputs the gut score acquired by the gut score acquisition unit.

16. A sound recording method for recording a user's abdominal sounds using the sound recording apparatus according to claim 1, comprising:
a first step of bringing the sound collecting portion into contact with the user's abdomen in a state where the user is in a sitting position;
a second step of causing the environment detection unit to perform the detection in a state where the sound collecting portion is in contact with the abdomen; and
a third step of causing the sound recording processing unit to perform processing related to the sound recording based on a result of detection by the environment detection unit.

17. A program that enables a computer of a sound recording apparatus that has a microphone accommodated in a housing thereof and is used to record a user's abdominal sounds in a state where a sound collecting portion of the housing near the microphone is in contact with the user's abdomen, to function as:
an environment detection unit that detects at least one of an inappropriate environment that is inappropriate for recording the abdominal sounds and an appropriate environment that is appropriate for recording the abdominal sounds; and
a sound recording processing unit that performs processing related to the sound recording based on a result of detection by the environment detection unit.
